# EUROPÄISCHE PATENTSCHRIFT

(11) **EP 2 351 736 B1**
(45) Veröffentlichungstag und Bekanntmachung des Hinweises auf die Patenterteilung: **06.03.2013**
(21) Anmeldenummer: 11163456.4
(22) Anmeldetag: 09.03.2009
(51) Int. Cl.: C14C 3/06, C07C 321/30, C14C 9/00, C07C 323/12, C07C 323/20, C07C 323/52, C07C 323/00

(54) **Lösungen von Thioethern und ihre Verwendung zur Herstellung von Leder**
Solutions of thioethers and their use in the production of leather.
Solutions de thioéthers et leur utilisation dans la fabrication de cuir

(30) Priorität: 13.03.2008 EP 08152701
(43) Veröffentlichungstag der Anmeldung: 03.08.2011
(62) Teilanmeldung aus: 09720794.8
(73) Patentinhaber: BASF SE, 67056 Ludwigshafen (DE)
(72) Erfinder: Hüffer, Stephan, 80639, München (DE); Garnier, Sebastien, 12161, Berlin (DE); Bette, Virginie, 68199, Mannheim (DE); Bohres, Edward, 67067, Ludwigshafen (DE); Wolf, Gerhard, 68775, Ketsch (DE); Annen, Ulrich, 67454, Haßloch (DE); Glocknitzer, Franz, 67480, Edenkoben (DE)

(56) Entgegenhaltungen:
- WO-A1-2007/063047
- US-A1- 2007 027 243
- DATABASE WPI Week 198844 Thomson Scientific, London, GB; AN 1988-310733 XP002642961, & JP 63 227566 A (IDEMITSU PETROCHEM CO) 21. September 1988 (1988-09-21)

## Beschreibung

Die vorliegende neue Lösungen von Thioethern und deren Verwendung .

Die Herstellung von Ledern mit geringer Neigung zum Vergilben, auch nach längerer Verwendungszeit, ist eine Aufgabe, die immer mehr Aufmerksamkeit genießt. Vergilbende Leder sehen nicht nur unästhetisch aus, auch ihre mechanischen Eigenschaften lassen in der Regel stark nach. Für die Vergilbungsneigung werden verschiedene Lederhilfsmittel verantwortlich gemacht, beispielsweise die Gerbmittel, So ist bekannt, dass viele Polykondensatgerbstoffe die Vergilbung von Leder nicht verhindern können.

Auch die Wahl der Fettungsmittel kann die Vergilbungsneigung beeinflussen.

Die Herstellung von Cr(VI)-armen Ledern ist eine Herausforderung, der immer mehr Aufmerksamkeit geschenkt wird. Spuren von Cr(VI) können in Ledern entstehen, die mit Hilfe von Cr(III)-Verbindungen wie beispielsweise Cr₂(SO₄)₃·n H₂O gegerbt wurden. Aus Umwelt- und Gesundheitsgründen ist die Entstehung von Cr(VI) in Leder unerwünscht. Es hat bereits verschiedene Versuche gegeben, die Bildung von Cr(VI) in Leder zu vermeiden.

So gibt es zahlreiche Versuche, durch chromfreie Gerbung den Gehalt an Cr(III) im Leder und damit auch die Bildung von Cr(VI) vollkommen zu vermeiden. Derartige Leder lassen sich auch in einigen Fällen einsetzen. Chromfrei gegerbte Halbzeuge sind auch als "wet white" bekannt. Jedoch sind chromfrei gegerbte Leder den Ledern auf Basis von wet blue in vielen Fällen noch unterlegen, was die mechanischen Eigenschaften betrifft. Außerdem können viele Leder auf Basis von wet white mit Ledern auf Basis von Wet blue nicht mithalten, was die Langzeitvergilbung betrifft, so dass noch immer mehr als 85% der Leder weltweit auf Basis von chromhaltige Gerbstoffen hergestellt werden.

Es wurde vorgeschlagen, L-Ascorbinsäure oder D-Isoascorbinsäure nach der Chromgerbung zuzusetzen, s. WO 2007/063047. Die Wirkung von L-Ascorbinsäure und D-Isoascorbinsäure ist jedoch nicht langfristig. Außerdem kann L-Ascorbinsäure die Analytik von Cr(VI) beeinflussen, indem Ascorbinsäure als Ligand für Cr(VI) wirkt.

Es wurde weiterhin vorgeschlagen, Ethylendiamintetraessigsäure (EDTA) bei der Chromgerbung zuzusetzen. Jedoch kann auch EDTA Cr(VI) maskieren. Außerdem sind EDTA-haltige Abwässer unerwünscht, weil EDTA Schwermetalle aus Schlämmen mobilisieren kann.

DE 198 60 610 schlägt vor, bei der Fettung ein oder mehrere Antioxidantien zuzusetzen, beispielsweise Ascorbinsäure, Bisphenolderivate oder Di-n-Butylditrtiocarbamate (Seite 2, Zeile 55). Die Langzeitwirkung derartiger Verbindungen ist jedoch für manche Zwecke noch nicht gut genug, insbesondere lässt in Langzeittests die Wärmevergilbung noch zu wünschen übrig.

DE 100 28 142 schlägt ebenfalls vor, Antioxidantien während des Pickelns oder während der Gerbung mit Cr(III)-Verbindungen einzusetzen, und nennt sterisch gehinderte Phenole wie 4,4'-Methylenbis-(2,6-di-tert.-butylphenol). Auch hier lässt sich die Langzeitwirkung noch verbessern.

Es wurde weiterhin vorgeschlagen, Gallussäure bei der Vor- oder Nachgerbung einzusetzen. Problematisch ist jedoch, dass Gallussäure zusammen mit Eisensalzen tiefschwarze Tinten geben kann. Eisenverbindungen sind in der Gerbung stets zugegen, beispielsweise aufgrund der Abnutzung der Spaltmaschinen. Man erhält beim Einsatz von Gallussäuren in der Gerbung oder Nachgerbung also schwarze oder dunkelgraue Leder, die sich nicht zur Herstellung von beispielsweise weißen Schuhen oder Möbelteilen eignen.

Es bestand also die Aufgabe, ein Verfahren zur Herstellung von Leder bereit zu stellen, das auch über einen längeren Zeitraum einen geringen Gehalt von Cr(VI) und außerdem über einen längeren Zeitraum sehr gute mechanische Eigenschaften bei gleichzeitig geringer Neigung zur Vergilbung aufweist. Weiterhin bestand die Aufgabe, Leder bereit zu stellen, die auch über einen längeren Zeitraum einen geringen Gehalt von Cr(VI) und außerdem über einen längeren Zeitraum sehr gute mechanische Eigenschaften bei gleichzeitig geringer Neigung zur Vergilbung aufweisen. Weiterhin bestand die Aufgabe, Schuhe, Möbel und Automobilinnenteile bereit zu stellen, die auch über einen längeren Zeitraum einen geringen Gehalt von Cr(VI) und außerdem über einen längeren Zeitraum sehr gute mechanische Eigenschaften bei gleichzeitig geringer Neigung zur Vergilbung aufweisen.

Dementsprechend wurden die eingangs definierten Lösungen gefunden.

Unter Cr(III)-haltigen Halbzeugen werden im Rahmen der vorliegenden Erfindung Zwischenprodukte in der Lederherstellung verstanden, die auf einer vorbehandelten Tierhaut, beispielsweise einer Haut von einem Säugetier wie Rind, Kalb, Schwein, Wird, Ziege oder Antilope, einem Fisch wie beispielsweise Aal, einem Vogel wie beispielsweise einem Strauß oder einem Reptil wie beispielsweise einer Schlange, basieren und die neben einigen Vorbehandlungsschritten auch mindestens einem Behandlungsschritt mit einer Cr(III)-Verbindung unterworfen worden sind, bei denen Cr(III)-Verbindungen in die Tierhaut eingelagert werden. Beispiele für derartige Behandlungsschritte mit einer Cr(III)-Verbindung sind Vorgerbung, Gerbung oder auch ein Pickel mit mindestens einer Cr(III)-Verbindung. Dabei ist es nicht erforderlich, dass Cr(III)-Verbindung in dem betreffenden Behandlungsschritt die einzige gerbend wirkende Verbindung ist, es kann sich auch um einen Behandlungsschritt mit einem Kombinationsgerbstoff oder einer Kombination von Gerbstoffen wie beispielsweise einer Mischung aus Cr(III)-Verbindung und einem Syntan, einem Polymergerbstoff oder einem Aldehyd wie beispielsweise Glutardialdehyd handeln.

Ein Gegenstand der vorliegenden Erfindung sind Lösungen von Thioether mit mindestens 10 C-Atomen, gewählt aus Thioethern der allgemeinen Formel I 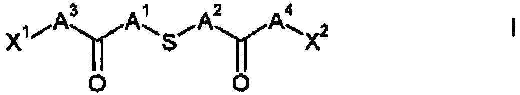 und Thioethern der allgemeinen Formel II 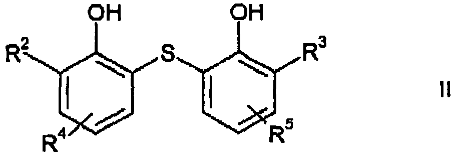 in einem Lösungsmittel, gewählt aus Glycerin oder Oligomeren von Ethylenglykol, insbesondere Triethylenglykol, oder einem Gemisch von Lösungsmitteln, enthaltend Glycerin oder ein oder mehrere Oligomere von Ethylenglykol, wobei die Variablen wie folgt definiert sind:
- X¹ und X²: verschieden oder gleich sind und gewählt aus C₁₀-C₄₀-Alkyl, C₁₀-C₄₀-Alkoxy, C₆-C₁₄-Aryl, C₆-C₁₄-Hydroxyaryl, jeweils unsubstituiert oder ein- oder mehrfach substituiert mit C₁-C₄-Alkyl, und stickstoffhaltigen Heterocyclen,
- A¹, A², A³ und A⁴: verschieden sind oder gleich und gewählt aus Spacem der Formel (CHR¹)ₙ, wobei
- R¹: gewählt wird aus C₁-C₄-Alkyl oder Wasserstoff und n für Spacer A³ und A⁴ eine ganze Zahl im Bereich von null bis 10 ist, und für Spacer A¹ und A² eine ganse Zahl von 1-10 ist
- R², R³: gleich oder verschieden und gewählt aus iso-Amyl, tert.-Amyl, iso-Propyl, tert.-Butyl und sec.-Butyl,
- R⁴, R⁵: gleich oder verschieden und gewählt aus Wasserstoff, Phenyl, Benzyl und C₁-C₄-Alkyl.

Erfindungsgemäße Lösungen können beispielsweise eine Konzentration von 0,1 bis 20 Gew.-% Thioether der allgemeinen Formel I oder II aufweisen, bevorzugt 0,5 bis 5 Gew.-%.

Dabei können solche erfindungsgemäße Lösungen, die ein Gemisch von Lösungsmitteln umfassen, ein oder mehrere von Oligomeren von Ethylenglykol und Glycerin verschiedene Lösungsmittel enthalten, beispielsweise Wasser. Erfindungsgemäße Lösungen können in einer Variante Mischungen von mehreren Oligomeren von Ethylenglykol enthalten, beispielsweise Mischungen von Diethylenglykol und Triethylenglykol oder Mischungen von Triethylenglykol und Tetraethylenglykol.

In einer Ausführungsform der vorliegende Erfindung enthalten vorstehend genannte Gemische mindestens 40 Gew.-% Oligomer von Ethylenglykol oder mindestens 20 Gew.-% Glycerin, bezogen auf erfindungsgemäße Lösung.

Erfindungsgemäße Lösungen lassen sich besonders gut zur Herstellung von Leder verwenden, insbesondere zur Durchführung des eingangs definierten erfindungsgemäßen Verfahrens.

Die Erfindung wird durch Beispiele erläutert.

Es wurden die folgenden Thioether, 1.2 und II.1 eingesetzt:

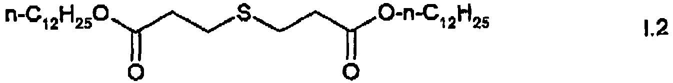

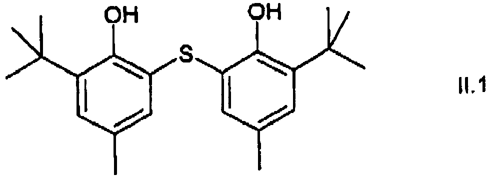

Als vergleichssubstanzen wurden die oxidierbaren Verbindungen V-Phenol.1 bis V-Phenol.3 eingesetzt

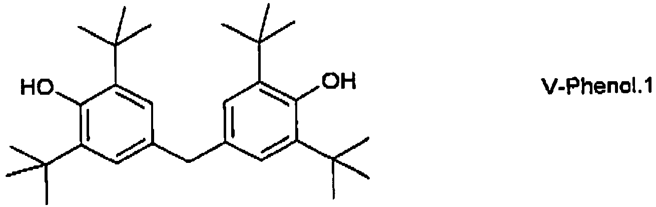

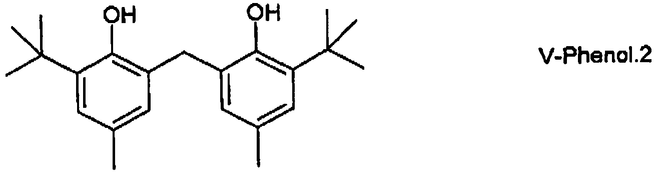

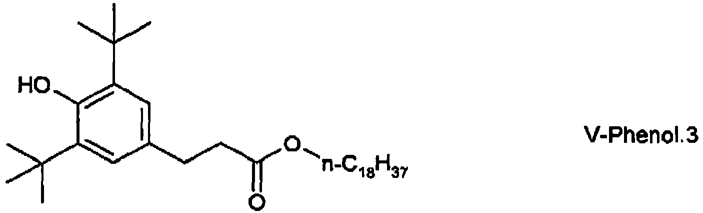

Thioether 1.2 und II.1, und V-Phenol.1 bis V-Phenol.3 wurden nach Literaturvorschriften hergestellt bzw. sind kommerziell erhältlich.

In den Beispielen wurde kein Farbstoff verwendet, um die Vergilbung leichter messen zu können. Man kann die Beispiele jedoch auch durchführen unter Verwendung von einem oder mehreren Lederfarbstoffen.

Man behandelte erfindungsgemäß bzw. in Vergleichsversuchen nach der folgenden allgemeinen Vorschrift:
Angaben in % sind stets Gew.-% und beziehen sich auf das Falzgewicht. Die Angaben in % bzw. Gew.-% betreffen bei wässrigen Formulierungen stets den Feststoff- bzw. Wirkstoffanteil, wenn nicht ausdrücklich anders angegeben.

Ein Rinder-wet blue (US-Packer) wurde auf 2,2 mm gefalzt und auf den Kerbereich beschnitten. Anschließend wurde in einem Fass neutralisiert, indem man bei 20°C und einer Flottenlänge von 100 Gew.-% Wasser, 1 Gew.-% Natriumformiat und 0,5 Gew.-% Natriumbicarbonat über eine Zeit von 60 Minuten (unter Walken) einwirken ließ, bis ein pH-Wert von 5 bis 5,5 erreicht war. Anschließend wurden die wet blues zweimal mit je 100 % Wasser gewaschen und in acht Streifen zu je ca. 600 g geschnitten. Die Streifen wurden auf acht separate Fässer verteilt und wie folgt weiter behandelt.

Man beaufschlagte die Fässer mit je 100 % Wasser sowie mit 4% des Sulfongerbstoffs aus EP-B 0 459 168, Beispiel K1, versetzt. Nach einer Vorlaufzeit von 20 Minuten dosierte man in Folge 3 % einer 2,5 Gew.-% Thioether, 1.2 oder 11.1, oder V-Phenol.1 bis V-Phonol.3-Lösung bzw. Dispersion in Triethylenglykol und danach jeweils 10 Gew.-% eines Fettlickers nach WO 03/023069, Beispiel A dosiert (Basis sulfitiertes Fischöl). Im achten Fass gab man weder Thioether noch Verbindung III.1 noch Vergleichssubstanz zu.

Anschließend walkte man 120 Minuten bei 30°C und säuerte mit 10% Ameisensäure auf einen pH-Wert der Flotte von 3,3 ab. Nach weiteren 20 Minuten ließ man die Flotte ab und wusch zweimal mit 100% Wasser. Die Leder werden über Nacht und mit einer Folie überdeckt auf Bock gelegt und dann bei 65°C für 4 Minuten im Vakuum getrocknet. Die Konditionierung vor den weiteren physikalisch-chemischen Tests erfolgte bei 40°C im Klimaraum über Nacht oder alternativ durch Einlegen in befeuchteten Sägespänen. Man erhielt die erfindungsgemäßen Leder L.1 bis L.3 und die Vergleichsleder V-L.4 bis V-L.8. Für die weiteren Tests wurden die Lederstreifen in entsprechend kleinere Stücke geteilt.

Die Fakra-Prüfung, eine Prüfung auf kurzfristige Vergilbungsstabilität, erfolgt gemäß DIN EN ISO 105-B06h bei 100°C unter Belichtung mit einer Hg-Lampe. Die Wärmevergilbung entsprechend Prüfverfahren bei 80°C wurde nach einem, drei, fünf und sieben Tagen in einem Notensystem von 1 bis 5 bewertet. Jeher höher die Note, desto geringer die Neigung zur Vergilbung bzw. Wärmevergilbung. Note 5 bedeutet keine visuelle Verschlechterung/Vergilbung im Vergleich zur Lederprobe vor Beginn des Tests.

**Tabelle 1: Vergilbungstests an erfindungsgemäßen Lederproben und an Vergleichsleder**

| Beispiel | FAKRA | Wärmevergilbung 80°C/1d | Wärmevergilbung 80°C/3d | Wärmevergilbung 80°C/5d | Wärmevergilbung 80°C/7d |
|---|---|---|---|---|---|
| L.2 | 4 | 3 | 2 | 2 | 1 |
| L.3 | 4 | 5 | 5 | 4 | 4 |
| V-L.5 | 2 | 2 | 2 | 1 | 1 |
| V-L.6 | 2 | 2 | 2 | 2 | 1 |
| V-L.7 | n.b. | 3 | 2 | 2 | 1 |
| V-L.8 | 2 | 2 | 2 | 1 | 1 |

| | | | | | |
|---|---|---|---|---|---|
| n.b.: nicht bestimmt | | | | | |

**Tabelle 2: Cr(VI)-Bestimmung an erfindungsgemäßen Lederproben und an Vergleichsleder**

| Beispiel | Cr(VI) nach 1d/80°C [ppm] | Cr(VI) nach 3d/80°C [ppm] | Cr(VI) nach 7d/80°C [ppm] |
|---|---|---|---|
| L.2 | 9 | n.b. | n.b. |
| L.3 | <3 | <3 | 7 |
| V-L.5 | 14 | 26 | 49 |
| V-L.6 | 11 | 24 | 45 |
| V-L.7 | 12 | 19 | 31 |
| V-L.8 | 32 | 48 | 69 |

| | | | |
|---|---|---|---|
| n.b.: nicht bestimmt | | | |

Aus erfindungsgemäßem Leder L.3 lässt sich Schuh.3 mit ausgezeichneten Gebrauchseigertschaften herstellen. Aus erfindungsgemäßem Leder L.2 lässt sich Schuh.2 mit sehr guten Gebrauchseigenschaften herstellen. Die erfindungsgemäßen Schuhe Schuh 2 bis Schuh 3 zeigen äußerst geringe bzw. nicht messbare Neigung zum Vergilben. Auch sind die Cr(VI)-Gehalte der erfindungsgemäßen Schuhe Schuh 2 bis Schuh 3 nicht nachweisbar.

## Patentansprüche

1. Lösung von Thioether mit mindestens 10 C-Atomen pro Molekül, gewählt aus Thioethern der allegemeinen Formel 1 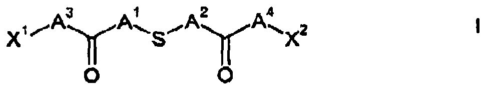 und Thioethern der allgemeinen Formel II 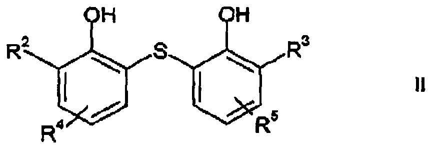 in einem Lösungsmittel, gewählt aus Glycerin oder Oligomeren von Ethylenglykol, oder einem Gernisch von Lösungsmitteln, enthaltend Glycerin oder ein oder mehrere Oligomere von Ethylenglykol, wobei die Variablen wie folgt definiert sind:
X¹ und X² verschieden oder gleich sind und gewählt aus C₁₀-C₄₀-Alkyl. C₁₀-C₁₀-Alkoxy, C₈-C₁₄-Aryl, C₆-C₁₄-Hydroxycyaryl, jeweils unsubstituiert oder ein- oder mehrfach substituiert mit C₁-C₄-Alkyl, und stickstoffhaltigen Heterocyclen,
A¹, A², A³ und A⁴ verschieden sind oder gleich und gewähit aus Spacern der Formel (CHR¹)ₙ, wobei
R¹ gewählt wird aus C₁-C₄-Alkyl oder Wasserstoff und n für Spacer A¹ und A² eine ganze Zahl im Bereich von 1 bis 10 ist und n für Spacer A³ und P⁴ eine ganze Zahl im Bereich von 0 bis 10 ist,
R², R³ gleich oder verschieden und gewählt aus iso-Amyl, tert-Amyl, iso-Propyl, tert.-Butyl und sec.-Butyl,
R⁴, R⁵ gleich oder verschieden und gewählt aus Wasserstoff, Phenyl, Benzyl und C₁-C₄-Alkyl.

2. Verwendung von Lösungen nach Anspruch 1 zur Herstellung von Leder aus Cs(III)-haltigen Halbzeugen.

## Claims

1. A solution of a thioether having at least 10 carbon atoms per molecule, selected from thioethers of the general formula I 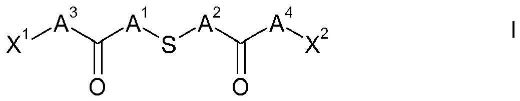 and thioethers of the general formula II 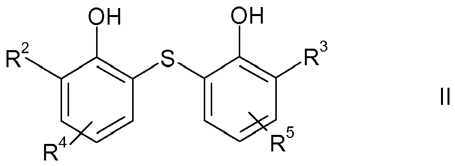 in a solvent selected from glycerol or oligomers of ethylene glycol, or a mixture of solvents comprising glycerol or one or more oligomers of ethylene glycol, the variables being defined as follows:
X¹ and X² are different or identical and are selected from C₁₀-C₄₀-alkyl, C₁₀-C₄₀-alkoxy, C₆-C₁₄-aryl, C₆-C₁₄-hydroxyaryl, in each case unsubstituted or mono- or polysubstituted by C₁-C₄-alkyl, and nitrogen-containing heterocycles,
A¹, A², A³ and A⁴ are different or identical and are selected from spacers of the formula (CHR¹)ₙ, where
R¹ is selected from C₁-C₄-alkyl or hydrogen and n for spacers A¹ and A² is an integer in the range from 1 to 10 and n for spacers A³ and A⁴ is an integer in the range from 0 to 10,
R², R³ are identical or different and are selected from isoamyl, tert-amyl, isopropyl, tert-butyl and sec-butyl,
R⁴, R⁵ are identical or different and are selected from hydrogen, phenyl, benzyl and C₁-C₄-alkyl.

2. The use of a solution according to claim 1 for the production of leather from Cr (III)-containing semifinished products.

## Revendications

1. Solution de thioéther contenant au moins 10 atomes C par molécule, choisi parmi les thioéthers de formule générale I 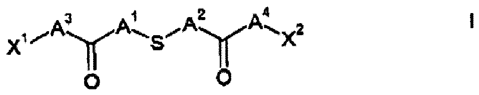 et les thioéthers de formule générale II 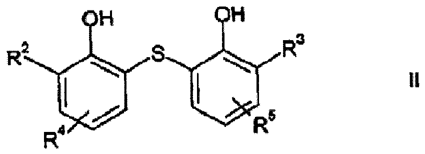 dans un solvant, choisi parmi la glycérine ou les oligomères d'éthylène glycol, ou un mélange de solvants, contenant de la glycérine ou un ou plusieurs oligomères d'éthylène glycol, les variables étant définies de la manière suivante :
X¹ et X² sont identiques ou différents, et choisis parmi alkyle en C₁₀-C₄₀, alcoxy en C₁₀-C₄₀, aryle en C₆-C₁₄, hydroxyaryle en C₆-C₁₄, à chaque fois non substitués ou substitués une ou plusieurs fois avec alkyle en C₁-C₄, et hétérocycles azotés,
A¹, A², A³ et A⁴ sont identiques ou différents, et choisis parmi les espaceurs de formule (CHR¹)ₙ,
R¹ étant choisi parmi alkyle en C₁-C₄ ou hydrogène et
n étant pour les espaceurs A¹ et A² un nombre entier dans la plage allant de 1 à 10 et n étant pour les espaceurs A³ et A⁴ un nombre entier dans la plage allant de 0 à 10,
R² R³ sont identiques ou différents, et choisis parmi iso-amyle, tert.-amyle, iso-propyle, tert.-butyle et sec.-butyle,
R⁴, R⁵ sont identiques ou différents, et choisis parmi hydrogène, phényle, benzyle et alkyle en C₁-C₄.

2. Utilisation de solutions selon la revendication 1 pour la fabrication de cuir à partir de produits semi-finis contenant Cr(III).
